# EUROPÄISCHE PATENTANMELDUNG

(11) **EP 4 706 701 A2**
(43) Veröffentlichungstag der Anmeldung: **11.03.2026**
(21) Anmeldenummer: 26153339.2
(22) Anmeldetag: 04.10.2016
(51) Int. Cl.: A61M 1/00

(54) **PUMPE, INSBESONDERE BLUTPUMPE**

(30) Priorität: 09.10.2015 EP 15189241
(62) Teilanmeldung aus: 19185610.3
(71) Anmelder: ECP Entwicklungsgesellschaft mbH, 52074 Aachen (DE)
(72) Erfinder: SIESS, Thorsten, 52074 Aachen (DE); SCHECKEL, Mario, 52074 Aachen (DE); SCHUMACHER, Jörg, 52074 Aachen (DE)
(74) Vertreter: Pfenning, Meinig & Partner mbB

(57) **Zusammenfassung**

Die Anmeldung betrifft eine Pumpe, insbesondere eine Blutpumpe. Die Pumpe umfasst eine in einer axialen Richtung verlaufende Antriebswelle (3), ein Förderelement (6), das in einem distalen Bereich der Antriebswelle (3) mit dieser verbunden ist und ein Gehäuse (5), welches das Förderelement (6) umgibt. Das Förderelement {6} und das Gehäuse (5) sind derart ausgebildet, dass diese sich nach einer aufgezwungenen Kompression selbstständig entfalten. Außerdem weist das Gehäuse (5) einen Einlassbereich (22) mit mindestens einer Einlassöffnung (23), einen flüssigkeitsdichten Bereich (20), der einen Bereich des Förderelements (5) umgibt, und einen Auslassbereich (24) mit mindestens einer Öffnung (23) zum Austritt eines Pumpmediums auf. Das Förderelement (6) ist derart angeordnet, dass es in den Auslassbereich (24) hineinragt.

## Beschreibung

Die Anmeldung betrifft eine Pumpe, insbesondere eine Blutpumpe, gemäß dem Oberbegriff des Anspruchs 1.

Aus dem Stand der Technik sind Blutpumpen mit einem proximalen und einem distalen Ende sowie einem dazwischen angeordneten Katheter bekannt, bei denen eine flexible Antriebswelle in einem Innenraum des Katheters geführt wird. Solche Blutpumpen weisen an ihrem distalen Ende typischerweise einen Pumpenkopf auf, der ein faltbares Gehäuse und ein faltbares Förderelement umfasst, wobei das Förderelement mit einem distalen Bereich der Antriebswelle verbunden ist. Das faltbare Gehäuse kann eine elastische Bespannung aufweisen. Derartige Pumpenköpfe können an schwer zugängliche Orte geführt werden. Beispielsweise kann ein solcher Pumpenkopf durch die Femoralarterie über den Aortenbogen in einen Bereich der Aortenklappe eines Patienten eingeschoben werden, um dort Blut vom linken Ventrikel des Herzens in die Aorta zu befördern. Angetrieben wird die Antriebswelle am proximalen Ende der Blutpumpe durch einen Motor, welcher sich typischerweise außerhalb des Körpers des Patienten befindet. Eine derartige Blutpumpe ist beispielsweise in der Druckschrift EP 2 868 331 A2 beschrieben.

Aufgabe der Anmeldung ist es, eine verbesserte Pumpe, insbesondere eine verbesserte Blutpumpe, vorzuschlagen, die effizienter im Betrieb ist.

Gelöst wird diese Aufgabe durch eine Pumpe mit den Merkmalen des Hauptanspruchs. Vorteilhafte Weiterbildungen ergeben sich mit den Merkmalen der abhängigen Ansprüche und der Ausführungsbeispiele.

Die vorgeschlagene Pumpe, insbesondere Blutpumpe, umfasst eine in einer axialen Richtung verlaufende Antriebswelle, ein Förderelement, das in einem distalen Bereich der Antriebswelle mit dieser verbunden ist und ein Gehäuse, welches das Förderelement umgibt. Das Förderelement und das Gehäuse sind derart ausgebildet, dass diese sich nach einer aufgezwungenen Kompression selbstständig entfalten. Außerdem weist das Gehäuse einen Einlassbereich mit mindestens einer Einlassöffnung, einen flüssigkeitsdichten Bereich, der einen Bereich des Förderelements umgibt, und einen Auslassbereich mit mindestens einer Öffnung zum Austritt eines Pumpmediums auf. Das Förderelement ist derart angeordnet, dass es in den Auslassbereich hineinragt.

Insbesondere kann es vorgesehen sein, dass das Förderelement in einem expandierten Zustand des Gehäuses und des Förderelements derart angeordnet ist, dass es in den Auslassbereich hineinragt. Der Auslassbereich umgibt einen in Förderrichtung angeordneten Bereich des Förderelements. Durch die mindestens eine Öffnung des Auslassbereichs ist das Förderelement im Auslassbereich nicht vollständig flüssigkeitsdicht umschlossen. Der Auslassbereich kann also einen Bereich des Förderelements umgeben, der an einem Ende des Förderelements gelegen ist, welches in Förderrichtung zeigt. Der flüssigkeitsdichte Bereich schließt sich typischerweise an ein in Förderrichtung gelegenes Ende des Einlassbereichs an und der Auslassbereich schließt sich typischerweise an ein in Förderrichtung gelegenes Ende des flüssigkeitsdichten Bereichs an. Typischerweise weisen der Einlassbereich, der flüssigkeitsdichte Bereich und der Auslassbereich zumindest bereichsweise einen im Wesentlichen kreisringförmigen Querschnitt auf. Es ist vorgesehen, dass das Pumpmedium im Betrieb der Pumpe durch den Einlassbereich in das Gehäuse hineinfließt und das Gehäuse über den Auslassbereich verlässt.

Das Gehäuse weist typischerweise im Auslassbereich zusätzlich zu axialen Öffnungen seitliche Öffnungen auf, so dass ein gefördertes Pumpmedium in dem Auslassbereich in radialer Richtung bzw. senkrecht zur Antriebswellenachse aus dem Gehäuse herausströmen kann oder beim Herausströmen zumindest eine radiale Geschwindigkeitskomponente aufweist.

Es hat sich überraschenderweise herausgestellt, dass durch eine derartige Anordnung die Pumpleistung im Vergleich zu bekannten Blutpumpen der oben beschriebenen Art drastisch erhöht werden kann. Beispielsweise kann ein in einem gegebenen Zeitintervall gefördertes Fluidvolumen auf diese Weise bei gleicher Motorleistung um bis zu 50% erhöht werden.

Typischerweise ragt hierbei das Förderelement teilweise aus dem flüssigkeitsdichten Bereich heraus. Das Förderelement ragt dann teilweise in den Auslassbereich hinein.

Es kann beispielsweise vorgesehen sein, dass der Auslassbereich sich mit mindestens 5%, bevorzugt mindestens 10%, besonders bevorzugt mindestens 25%, einer axialen Ausdehnung des Förderelements überlappt.

Weiterhin kann es vorgesehen sein, dass der Auslassbereich sich mit höchstens 75%, bevorzugt höchstens 65%, besonders bevorzugt höchstens 50%, einer axialen Ausdehnung des Förderelements überlappt.

Typischerweise umfasst das Gehäuse insbesondere im Auslassbereich ein Gitter. Das Gitter kann ein Formgedächtnis-Material oder eine geeignete Gedächtnislegierung umfassen, so dass das Gitter verlässlich expandier- und komprimierbar ist. Das Gitter kann beispielsweise Nitinol, einen Kunststoff, eine Eisenlegierung oder eine Kupferlegierung umfassen. Es kann vorgesehen sein, dass das Gehäuse im Einlassbereich und/oder im flüssigkeitsdichten Bereich und/oder im Auslassbereich ein Gitter umfasst.

Typischerweise weist das Gehäuse eine elastische Bespannung auf. Die elastische Bespannung kann beispielsweise auf einer Innenseite und/oder auf einer Außenseite eines gegebenenfalls vorhandenden Gitters angeordnet sein. Eine Bespannung ist geeignet, Gitteröffnungen zu verschließen. Hierbei kann es sich beispielsweise um eine Polyurethan-Bespannung handeln. Es können aber auch beispielsweise Polyethylen, Polypropylen, Silikon oder Parylene verwendet werden.

Der flüssigkeitsdichte Bereich kann zumindest teilweise durch die elastische Bespannung gebildet werden. Typischerweise werden der Auslassbereich und der Einlassbereich durch das Gitter mit seinen Gitteröffnungen gebildet, während zwischen dem Auslassbereich und dem Einlassbereich der flüssigkeitsdichte Bereich durch eine Bespannung des Gitters mit der elastischen Bespannung gebildet wird.

Es kann vorgesehen sein, dass das Gehäuse in einem expandierten Zustand im Auslassbereich einen sich in Förderrichtung verjüngenden konischen Abschnitt aufweist. Es kann auch vorgesehen sein, dass das Gehäuse in einem expandierten Zustand im Einlassbereich einen sich in Förderrichtung aufweitenden konischen Abschnitt aufweist.

Das Gehäuse kann außerdem in einem expandierten Zustand im Auslassbereich einen im Wesentlichen rohrförmigen Abschnitt aufweisen, der an einem in Förderrichtung gelegenen Ende mit dem konischen Abschnitt verbunden ist. Der rohrförmige Abschnitt des Auslassbereichs kann an einem der Förderrichtung entgegengesetzten Ende in einen rohrförmigen Abschnitt des Flüssigkeitsdichten Bereichs übergehen.

Typischerweise weist das Gitter in dem Auslassbereich größere Gitteröffnungen als in dem flüssigkeitsdichten Bereich auf. Außerdem kann vorgesehen sein, dass das Gitter in dem Einlassbereich größere Gitteröffnungen aufweist als in dem flüssigkeitsdichten Bereich. Durch eine Vergrößerung der Öffnungen in den durchströmten Bereichen des Gehäuses kann eine Blutschädigung durch die Pumpe verhindert oder zumindest minimiert werden.

Es kann vorgesehen sein, dass ein Bereich des Auslassbereichs von einem Abströmelement umgegeben ist. Insbesondere kann es vorgesehen sein, dass ein Bereich des Auslassbereichs von einem Abströmschirm umgeben ist, der sich vom Pumpengehäuse in Förderrichtung erstreckt. Dieser Abströmschirm kann im Wesentlichen eine Form aufweisen, die einer Mantelfläche eines Kegelstumpfes entspricht. Typischerweise ist ein verjüngtes Ende des Abströmschirms an dem Gehäuse, insbesondere im flüssigkeitsdichten Bereich, befestigt. Ein sich weitendes Ende des Abströmschirms kann in Förderrichtung ausgerichtet sein, so dass der Abströmschirm den Auslassbereich teilweise oder vollständig umschließt. Durch den Abströmschirm können die Strömungsbedingungen in der Pumpe weiter optimiert werden, wodurch die Förderleistung verbessert werden kann.

Es ist auch möglich, dass ein Bereich des Auslassbereichs von einem Abströmschlauch umgeben ist, der sich vom Pumpengehäuse in Förderrichtung erstreckt. Typischerweise ist der Abströmschlauch derart flexibel ausgebildet, dass dieser ein Rückschlagventil ausbildet. Ein solcher Abströmschlauch kann ähnlich wie der Abströmschirm zu einer weiteren Optimierung der Strömungsbedingungen und zu einer Verbesserung der Pumpleistung beitragen. Es ist beispielsweise ein Abströmschlauch, wie in der Druckschrift EP 2 345 440 B1 beschrieben, möglich.

Typischerweise überlappt der Einlassbereich sich nicht mit einer axialen Ausdehnung des Förderelements. Somit kann das Förderelement derart angeordnet sein, dass dieses nicht in den Einlassbereich hineinragt. Dadurch kann beispielsweise eine Abschirmung des Förderelements von Körperbestandteilen eines Patienten in einem Ansaugbereich der Pumpe erreicht werden, wodurch eine Schädigung des Patienten vermieden werden kann.

Typischerweise ist die Antriebswelle an einem proximalen Ende der Antriebswelle mit einem Motor zum Antreiben der Antriebswelle verbunden. Beispielsweise kann die Welle eine flexible Welle sein, die in einem Katheter geführt wird.

Die Pumpe kann eingerichtet sein zum Pumpen von Blut von einem Ventrikel in ein Blutgefäß eines Patienten, wobei die Antriebswelle in einem proximalen Bereich zum Anschließen an einen Motor außerhalb eines Körpers des Patienten eingerichtet ist. Der Motor kann beispielsweise für eine Befestigung an einem Oberschenkel eines Patienten eingerichtet sein. Zu diesem Zweck können der Katheter und die Antriebswelle eine ausreichende Länge von mindestens 50 cm, vorzugsweise mindestens 90 cm, aufweisen. Eine maximale Länge der flexiblen Antriebswelle kann 200 cm, vorzugsweise 150 cm, betragen.

Das Gitter kann in einem Bereich des Gehäuses als Rautengitter mit im Wesentlichen rautenförmigen Gitteröffnungen ausgebildet sein.

Es kann vorgesehen sein, dass das Gitter Gitterstreben aufweist, wobei eine Anzahl der Gitterstreben entlang eines Umfangs des Rautengitters m·2ⁿ mit natürlichen Zahlen m und n, vorzugsweise 32 oder 40, beträgt, wobei m größer als 2, vorzugsweise größer als 3, ist. Außerdem kann vorgesehen sein, dass das Gehäuse im Auslassbereich entlang eines Umfangs m, vorzugsweise 4 oder 5, Gitterstreben aufweist.

Eine solche Ausführung erlaubt eine besonders stabile Reduzierung der Gitterstreben, beispielsweise in Richtung eines distalen oder in Richtung eines proximalen Endes des Gehäuses. Darüber hinaus kann auf diese Weise eine besonders stabile Vergrößerung der Gitteröffnungen erreicht werden. Hierbei kann vorgesehen sein, dass alle Gitterstreben entlang eines Umfangs des Gehäuses an einer gewissen axialen Position in Form des Buchstabens Y paarweise zusammengeführt werden. Ein derartiges Zusammenführen kann wiederholt an einer oder mehreren weiteren axialen Positionen des Gehäuses erfolgen. Somit kann beispielsweise an einem Ende des Gehäuses bei einem vorliegen von beispielsweise 5 Streben und Öffnungen entlang des Umfangs des Gehäuses eine Schrittweise Erhöhung der Anzahl der Streben auf 10, 20, 40, ... erfolgen. Die Anzahl der Streben und Öffnungen kann beispielsweise auch angefangen von 4 (3) Streben und Öffnungen entlang des Umfanges des Gehäuses schrittweise auf 8 (6), 16 (12), 32 (24), ... erhöht werden. n bezeichnet hierbei eine Anzahl der Schritte und m eine Anzahl der Streben und Öffnungen entlang eines Umfangs des Gehäuses an einer axialen Position, bei der das Zusammenführen beginnt.

Die Anmeldung bezieht sich außerdem auf eine Blutpumpe, die eine in einer axialen Richtung verlaufende Antriebswelle, ein Förderelement, das in einem distalen Bereich der Antriebswelle mit dieser verbunden ist, und ein Gehäuse, welches das Förderelement umgibt, umfasst. Das Förderelement und das Gehäuse sind derart ausgebildet, dass diese sich nach einer aufgezwungenen Kompression selbstständig entfalten. Das Gehäuse weist ein Gitter, einen Einlassbereich mit mindestens einer Einlassöffnung, einen flüssigkeitsdichten Bereich, der einen Bereich des Förderelements umgibt, und einen Auslassbereich mit mindestens einer Öffnung zum Austritt eines Pumpmediums auf. Außerdem weist das Gitter Gitteröffnungen und Gitterstreben auf, wobei eine Anzahl der Gitterstreben entlang eines Umfangs des Gitters an einer ersten axialen Position des Gitters m beträgt und wobei das Gitter derart ausgeführt ist, dass sich die Anzahl der Gitterstreben entlang eines Umfangs des Gitters in einer axialen Richtung mit n Schritten auf m·2ⁿ Gitterstreben an einer zweiten axialen Position des Gitters erhöht, wobei m und n natürlichen Zahlen sind und m größer als 2, vorzugsweise größer als 3, ist. m kann beispielsweise 3, 4 oder 5 betragen. Alternativ kann m beispielsweise 6, 8 oder 10, betragen.

Typischerweise ist vorgesehen, dass die Anzahl der Gitterstreben entlang des Umfangs des Gitters an der zweiten axialen Position des Gitters 32 oder 40 beträgt. Außerdem ist typischerweise vorgesehen, dass das Gehäuse im Auslassbereich entlang eines Umfangs m, vorzugsweise 4 oder 5, Gitterstreben aufweist. Durch eine derartige Ausführung kann eine Blutschädigung beim Durchfließen durch die Gitterstreben des Auslassbereichs verringert werden.

Ausführungsbeispiele der Erfindung werden nachfolgend anhand der Abbildungen beschrieben. Es zeigen
- Fig. 1: eine schematische Darstellung einer Pumpenanordnung,
- Fig. 2: eine schematische Darstellung eines Pumpenkopfes,
- Fign. 3(a), (b): zwei weitere schematische Darstellungen des Pumpenkopfes,
- Fig. 4: eine schematische Darstellung eines Gehäuses,
- Fig. 5: eine schematische Darstellung eines Motors und
- Fig. 6: eine schematische Darstellung eines weiteren Motors.

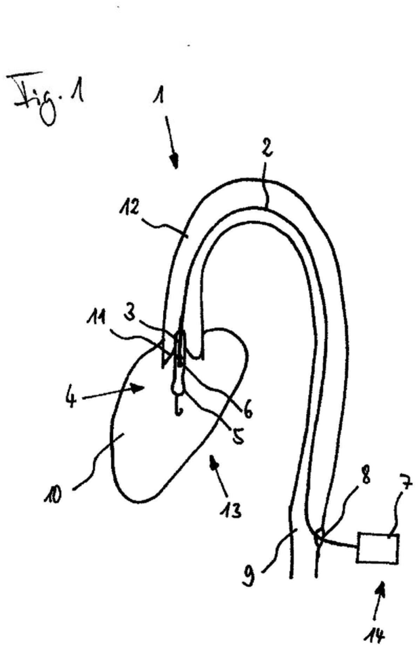

Fig. 1 zeigt schematisch eine Pumpenanordnung 1. Die Pumpenanordnung 1 umfasst einen Katheter 2, in welchem eine flexible Antriebswelle 3 geführt wird. Der Katheter 2 ist mit einem Pumpenkopf 4 verbunden. Dieser Pumpenkopf 4 umfasst ein Gehäuse 5 und ein in dem Gehäuse 5 angeordnetes Förderelement 6, welches über die Antriebswelle 3 mit einem am proximalen Ende der Antriebswelle 3 angeschlossenen Motor 7 angetrieben werden kann. Der Pumpenkopf 4 sowie der Katheter 2 und die Antriebswelle 3 werden über eine Schleuse 8 in die Femoralarterie 9 eingeführt, derart, dass sich der Pumpenkopf 4 im Bereich der linken Herzkammer 10 im Bereich der Aortenklappe 11 befindet. Im Betrieb wird die Antriebswelle 3 durch den Motor 7 angetrieben und die Pumpenanordnung 1 fördert Blut von der linken Herzkammer 10 in die Aorta 12. In der gezeigten Anordnung zur Linksherz-Unterstützung entspricht eine Förderrichtung der Pumpenanordnung 1 der Richtung von einem distalen Ende 13 der Pumpenanordnung 1 zu einem proximalen Ende 14 der Pumpenanordnung 1.

Die Pumpenanordnung 1 kann aber auch eingerichtet sein für ein Fördern von Blut in einer Richtung von dem proximalen Ende 14 zum distalen Ende 13 der Pumpenanordnung 1, was sich beispielsweise für eine Rechtsherz-Unterstützung eignet.

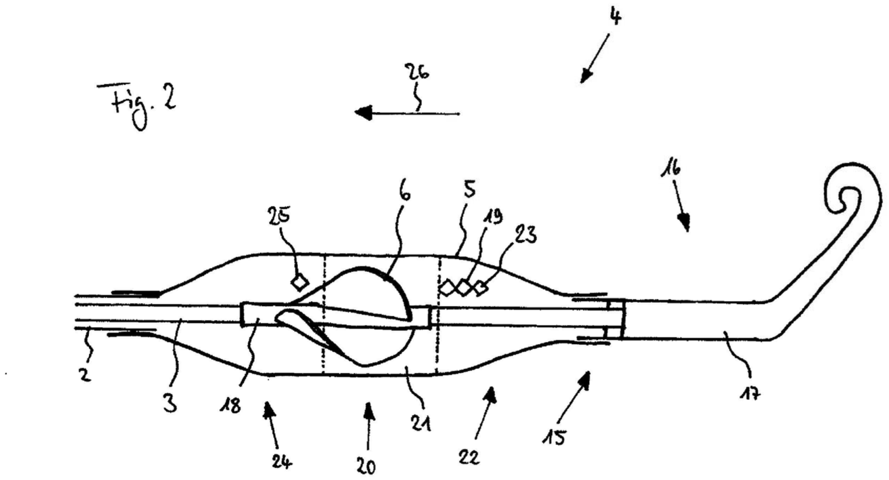

Der Pumpenkopf 4 ist in Fig. 2 schematisch dargestellt. Wiederkehrende Merkmale sind in dieser und in den nachfolgenden Abbildungen mit den gleichen Bezugszeichen versehen. Der Pumpenkopf 4 umfasst das Förderelement 6 und das Gehäuse 5. Das Förderelement 6 ist im vorliegenden Beispiel als Pumpenrotor mit zwei flexiblen Segmenten in Form von Rotorblättern ausgeführt. Zusätzlich ist die Antriebswelle 3 dargestellt, die an einem distalen Bereich 15 des Pumpenkopfes 4 gelagert ist. An einem distalen Ende 16 des Pumpenkopfes 4 ist ein sogenannter Pig-Tail 17 vorgesehen, der aus einem elastisch verformbaren Material gefertigt ist. Mit der Antriebswelle 3 ist ein zylindrisches Element 18 starr verbunden. Auf dem zylindrischen Element 18 ist das Förderelement 6 befestigt. Sowohl das Förderelement 6 als auch das Gehäuse 5 sind derart entfaltbar ausgeführt, dass diese sich nach einer aufgezwungenen Kompression selbstständig entfalten. Das Förderelement 6 ist aus einem Kunststoff gefertigt. Das Gehäuse 5 ist aus dem Formgedächtnismaterial Nitinol gefertigt. Dadurch, dass sowohl das Förderelement 6 als auch das Gehäuse 5 entfaltbar ausgebildet sind, ist der gesamte Pumpenkopf 4 entfaltbar.

Das Gehäuse 5 ist als Rautengitter 19 ausgeführt und weist in einem flüssigkeitsdichten Bereich 20 eine elastische Bespannung 21 aus Polyurethan auf. Die elastische Bespannung 21 bedeckt eine Innenseite und eine Außenseite des Rautengitters 19 derart, dass durch das Gitter 19 ausgebildete rautenförmige Gitteröffnungen in dem flüssigkeitsdichten Bereich 20 durch die elastische Bespannung 21 flüssigkeitsdicht verschlossen werden.

Darüber hinaus weist das Gehäuse 5 einen Einlassbereich 22 auf, der nicht von der elastischen Bespannung 21 bedeckt wird. In dem Einlassbereich 22 bilden die rautenförmigen Gitteröffnungen Einlassöffnungen aus, von denen eine in Fig. 2 beispielhaft mit dem Bezugszeichen 23 versehen ist. Außerdem weist das Gehäuse 5 einen Auslassbereich 24 auf, der ebenfalls nicht von der elastischen Bespannung 21 bedeckt wird. In dem Auslassbereich 24 bilden die rautenförmigen Gitteröffnungen Auslassöffnungen aus, von denen eine beispielhaft gezeigt und mit dem Bezugszeichen 25 versehen ist.

Bei einem Betrieb der Pumpenanordnung 1 wird die Antriebswelle 3 durch den Motor 7 angetrieben, so dass das mit der Antriebswelle 3 verbunden Förderelement 6 um eine Achse der Antriebswelle 3 rotiert. Dadurch wird Blut durch die Einlassöffnungen des Einlassbereichs 22 in das Gehäuse 5 befördert und tritt anschließend durch die Auslassöffnungen des Auslassbereichs 24 aus dem Gehäuse 5 aus. Auf diese Weise wird durch die Pumpenanordnung 1 Blut in einer Förderrichtung 26 gefördert.

Die elastische Bespannung 21 umgibt eine axiale Ausdehnung des Förderelements 6 nicht vollständig. Das Förderelement 6 ragt stattdessen teilweise in den Auslassbereich 24 hinein, so dass zumindest die Auslassöffnung mit dem Bezugszeichen 25 seitlich, d.h. in radialer Richtung, neben dem Förderelement 6 angeordnet ist. Die elastische Bespannung 21 ist hingegen an ihrem distalen Ende derart ausgeführt, dass das Förderelement 6 nicht oder nicht wesentlich in den Einlassbereich 22 hineinragt und somit nicht seitlich von Einlassöffnungen umgeben ist.

Die elastische Bespannung 21 und das Förderelement 6 sind so ausgestaltet und zueinander angeordnet, dass etwa ein Drittel der axialen Ausdehnung des Förderelements 6 nicht von der elastischen Bespannung 21, die den flüssigkeitsdichten Bereich 20 bildet, umgeben ist. Derselbe Anteil der axialen Ausdehnung des Förderelements 6 ist in dem gezeigten Beispiel von dem Auslassbereich 24 umgeben.

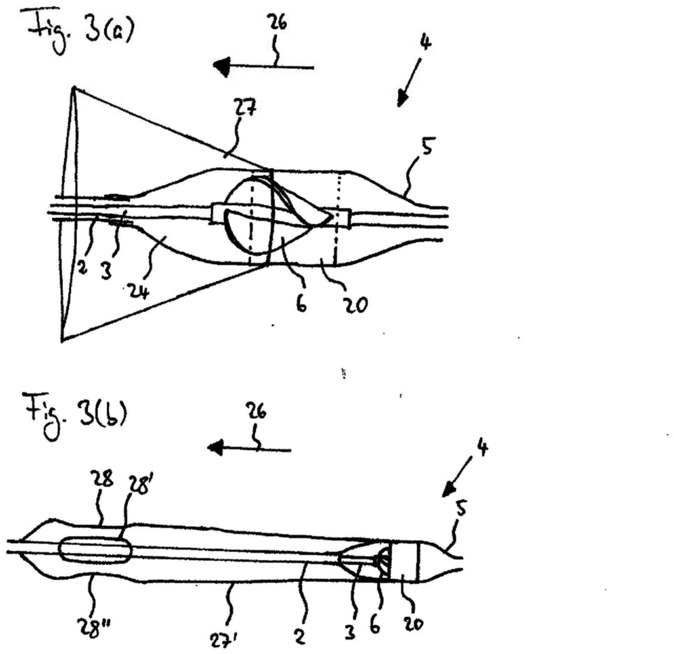

Zusätzlich umfasst der Pumpenkopf 4 ein Abströmelement. Dieses kann als Abströmschirm 27, wie in Fig. 3(a) dargestellt ist, oder als Abströmschlauch 27', wie in Fig. 3(b) dargestellt ist, ausgeführt sein.

Der in Fig. 3(a) dargestellte Abströmschirm 27 ist im flüssigkeitsdichten Bereich 20 des Gehäuses 5 am Gehäuse 5 befestigt. Der Abströmschirm 27 hat die Form eines Mantels eines Kegelstumpfes und erstreckt sich so in Förderrichtung 26, dass dieser in Förderrichtung 26 aufgeweitet ist. Durch den Abströmschirm 27 werden das Förderelement 6 und der Auslassbereich 24 umgeben. Es kann in einer anderen Ausführung auch vorgesehen sein, dass der Auslassbereich 24 durch den Abströmschirm 27 teilweise umgeben wird.

Der Pumpenkopf 4 in Fig. 3(b) unterscheidet sich von dem in Fig. 3(a) gezeigten Pumpenkopf 4 lediglich dadurch, dass anstatt des Abströmschirms 27 ein Abströmschlauch 27' vorgesehen ist. Dieser ist an dem Gehäuse 5 in dem flüssigkeitsdichten Bereich 20 befestigt und erstreckt sich von dort in Förderrichtung 26. Der Abströmschlauch 27' ist aus Polyurethan gefertigt und weist Öffnungen 28, 28', 28" in einem in Förderrichtung 26 gelegenen Bereich auf. In dem gezeigten Beispiel ist der Auslassbereich 24 vollständig von dem Abströmschlauch 27' umgeben. Der Abströmschlauch 27' ist flexibel und verschließt sich selbstständig, wenn eine Blutströmung entgegengesetzt zur Förderrichtung 26 auftritt, dadurch, dass der Abströmschlauch 27' an den Katheter 2 und/oder an das Gehäuse 5 gedrückt wird.

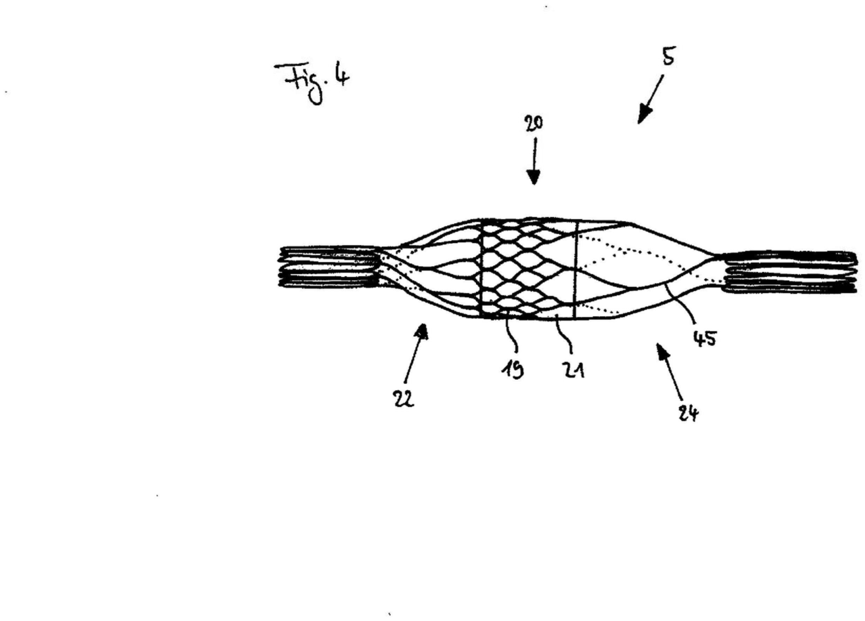

Fig. 4 zeigt schematisch das Rautengitter 19 des Gehäuses 5. Zusätzlich ist der flüssigkeitsdichte Bereich 20 mit der elastischen Bespannung 21 dargestellt sowie der Einlassbereich 22 und der Auslassbereich 24. Bereiche des Einlassbereichs 22 und des Auslassbereichs 24 weisen eine konische Form auf, während der flüssigkeitsdichte Bereich 20 im Wesentlichen rohrförmig ist. Das Gitter 19 weist Gitterstreben auf, von denen eine beispielshaft mit der Bezugsziffer 45 gekennzeichnet ist. Die Gitterstreben 45 verlaufen derart, dass die im Wesentlichen rautenförmigen Gitteröffnungen sowohl im Einlassbereich 22 als auch im Auslassbereich 24 größer sind als im flüssigkeitsdichten Bereich 20. Zur verbesserten Übersicht sind auf einer vom Betrachter abgewandten Seite des Gehäuses 5 angeordnete Gitterstreben in Fig. 4 lediglich gepunktet angedeutet.

Im flüssigkeitsdichten Bereich 20 bilden die Gitterstreben 45 ein vergleichsweise engmaschiges Gitter 19 aus. Entlang eines Umfangs des Gehäuses 5 im flüssigkeitsdichten Bereich 20 weist das Gitter 19 zweiunddreißig Streben beziehungsweise, sofern der Umfang an einer axialen Position des Gehäuses 5 mit Knotenpunkten betrachtet wird, sechzehn Knoten auf. Durch ein derart engmaschiges Gitter 19 wird ein weitgehend runder Querschnitt des Gehäuses 5 in dem flüssigkeitsdichten Bereich 20 erreicht.

Vom flüssigkeitsdichten Bereich 20 in Richtung des Einlassbereichs 22 und in Richtung des Auslassbereichs 24 wird die Anzahl der Gitterstreben 45 entlang eines Umfangs des Gehäuses 5 durch ein paarweises Zusammenführen der Gitterstreben halbiert, so dass das Gehäuse 5 in den entsprechenden Bereichen sechzehn Gitterstreben 45 entlang des Umfangs aufweist, in dem keine Knotenpunkte vorliegen. Anschließend wird die Anzahl der Gitterstreben 45 in Richtung des Einlassbereichs 22 und des Auslassbereichs 24 erneut durch das paarweise Zusammenführen der Gitterstreben 45 reduziert, so dass Gehäuse 5 in diesen Bereichen acht Gitterstreben 45 aufweist. Im Auslassbereich 24 erfolgt eine weitere Reduzierung der Anzahl der Gitterstreben 45 in der oben genannten Weise, so dass das Gehäuse 5 in einem weiter in Förderrichtung 26 gelegenen Bereich lediglich vier Gitterstreben 45 entlang eines Umfangs aufweist.

Durch die beschriebene Reduzierung der Anzahl der Gitterstreben 45 bildet sich in dem Einlassbereich 22 und in dem Auslassbereich 24 ein Gitter 19 mit größeren Gitteröffnungen als im flüssigkeitsdichten Bereich 20 aus.

Die Gitterstreben 45 bilden in den konischen Bereichen des Auslassbereichs 24 und des Einlassbereichs 22 eine spiralförmige Struktur, was bei einem Hinausschieben des Pumpenkopfes 4 aus einer Kanüle zu einem zu einem zuverlässigen Entfalten des Pumpenkopfs 4 führt.

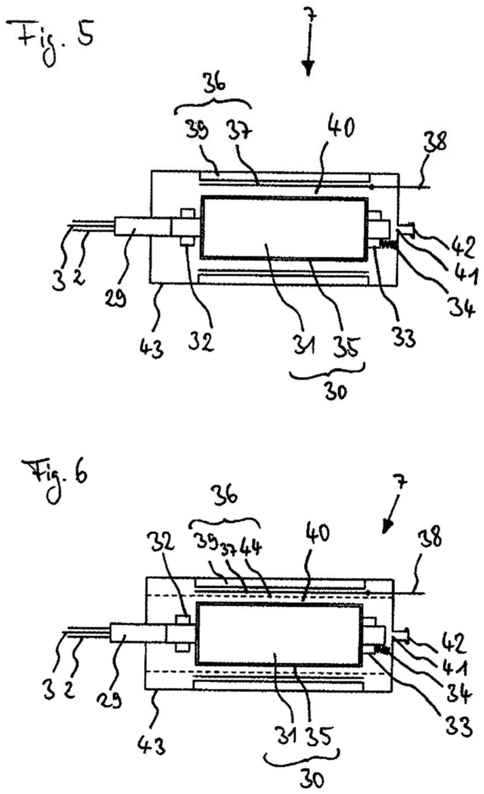

Fig. 5 zeigt eine schematische Ansicht des Motors 7. Der Motor 7 ist im Bereich eines Wellenstummels 29 mit dem Katheter 2 verbunden, der in den Wellenstummel 29 eingeklebt ist. In dem Katheter 2 wird die flexible Antriebswelle 3 geführt. Der Motor 7 weist außerdem einen Rotor 30 auf, der einen Rotormagneten 31 umfasst.

Die flexible Antriebswelle 3 ist derart mit dem Rotor 30 verbunden, dass ein Drehmoment bei einer Rotation des Rotors 30 von dem Rotor 30 auf die flexible Antriebswelle 3 übertragen wird. Über die flexible Antriebswelle wird das Drehmoment auf das Förderelement 6 übertragen, so dass die Pumpenanordnung 1 durch den Motor 7 angetrieben wird.

Der Rotor 30 ist axial durch zwei Lager 32, 33 gelagert. Eines dieser Lager 33 ist durch ein Federelement 34 vorgespannt für eine axiale Stabilisierung des Rotors 30. Das Federelement 34 kann beispielsweise als eine Schraubenfeder oder als eine Ringfeder ausgeführt sein. Die Lager 32, 33 können jeweils als Kugellager oder als Gleitlager ausgeführt sein. Wenn die Lager 32, 33 als Kugellager ausgeführt sind, umfassen die Lager 32, 33 Kugeln aus Keramik und Käfige aus Kunststoff, so dass die Kugellager nichtmagnetisierbares Material aufweisen. Die Ringe der Lager können beispielsweise aus einem magnetisierbaren Metall oder aus einem nichtmagnetisierbaren Material gefertigt sein. Wenn die Lager 32, 33 als Gleitlager ausgeführt sind, umfassen diese jeweils Gleitpartner aus DLC-beschichtetem Implantatstahl und Yttrium-stabilisiertem Zirconiumoxid.

Der Rotormagnet 31 weist eine biokompatible DLC-Beschichtung 35 auf. Außerdem weist der Motor 7 einen Stator 36 auf. Der Stator 36 umfasst mehrere Wicklungen 37, die mit Stromanschlüssen 38 elektrisch leitfähig verbunden sind. Darüber hinaus weist der Stator 36 Rückschlussbleche 39 auf. Die Wicklungen 37 sind mit einem biokompatiblen Epoxidharz vergossen, welches wärmeleitendes Aluminiumoxid enthält.

Zwischen einer Innenseite der Beschichtung der Wicklungen 37 und einer Außenseite der Beschichtung 35 des Rotormagneten 31 ist ein Spalt 40 mit ringförmigem Querschnitt ausgebildet. Der Spalt 40 weist eine Breite von 0,2 mm auf. Dieser Spalt 40 steht in fluider Verbindung mit einer Spülöffnung 41, die mit einem Spülanschluss 42 verbunden ist, wobei der Spülanschluss 42 an einem proximalen Ende des Motors 7 angeordnet ist. Darüber hinaus steht der Spalt 40 in fluider Verbindung mit einem zwischen der Antriebswelle 3 und dem Katheter 2 ausgebildeten Zwischenraum. Somit kann beispielsweise eine Glukoselösung über den Spülanschluss 42 durch die Spülöffnung 41 und den Spalt 40 und den Zwischenraum gespült werden. Auf diese Weise wird der Rotor 30 im Betrieb von der Glukoselösung umspült. Ein radialer Abstand zwischen einer Außenseite des Rotormagneten 31 und einer Innenseite der Wicklungen 37 beträgt 0,5 mm. Ein Innenradius der Wicklungen 37 entspricht hierbei dem 1,1-fachen eines Außenradius des Rotormagneten 31.

Der Stator 36 und der Rotor 30 sind für einen Anwender nicht lösbar miteinander verbunden und in ein Motorgehäuse 43 eingefasst. Das Motorgehäuse 43 kann beispielsweise mit einem Griff oder einem Kühlkörper verbunden sein. Durch den geringen Abstand zwischen den Wicklungen 37 und dem Rotormagneten 31 kann der Motor sehr effizient betrieben werden, so dass sowohl das Motorgehäuse 43 als auch ein gegebenenfalls mit diesem verbundener Griff oder Kühlkörper an seinen berührbaren Oberflächen auf weniger als 40 °C erwärmt wird, wenn die Pumpenanordnung 1 bei einer Drehzahl von 32.000 Umdrehungen pro Minute und einer Förderleistung von 2.5 I pro Minute betrieben wird.

Der in Fig. 6 dargestellte Motor 7' unterscheidet sich von dem in Fig. 6 dargestellten Motor 7 lediglich dadurch, dass der Stator 36 in dieser Ausführung eine flüssigkeitsdichte Hülse 44 aufweist, die den Spalt 40 begrenzt. In dieser Ausführung beträgt die Breite des Spalts 40 0,15 mm. Die Hülse 44 umfasst Polyetheretherketon und ist magnetisch inaktiv. Die Hülse 44 ist derart angeordnet, dass beispielsweise die Wicklungen 37 und weitere Teile des Stators 36 durch die Hülse 44 von der gegebenenfalls durch den Spalt 40 fließenden Spülflüssigkeit getrennt werden. Eine Ausdehnung der Hülse 44 in axialer Richtung beträgt etwa das 1,2-fache einer axialen Ausdehnung des Rotormagneten 31.

Lediglich in den Ausführungsbeispielen offenbarte Merkmale der verschiedenen Ausführungsformen können miteinander kombiniert und einzeln beansprucht werden.

Die vorliegende Anmeldung bezieht sich unter anderem auf die folgenden Aspekte:
1. Pumpe, insbesondere Blutpumpe, umfassend eine in einer axialen Richtung verlaufende Antriebswelle (3), ein Förderelement (6), das in einem distalen Bereich der Antriebswelle (3) mit dieser verbunden ist, ein Gehäuse (5), welches das Förderelement (6) umgibt, wobei das Förderelement (6) und das Gehäuse (5) derart ausgebildet sind, dass diese sich nach einer aufgezwungenen Kompression selbstständig entfalten, und wobei das Gehäuse (5) einen Einlassbereich (22) mit mindestens einer Einlassöffnung (23), einen flüssigkeitsdichten Bereich (20), der einen Bereich des Förderelements (6) umgibt, und einen Auslassbereich (24) mit mindestens einer Öffnung (23) zum Austritt eines Pumpmediums aufweist,
   dadurch gekennzeichnet, dass
   das Förderelement (6) derart angeordnet ist, dass es in den Auslassbereich (24) hineinragt.
2. Pumpe nach Aspekt 1, dadurch gekennzeichnet, dass der Auslassbereich (24) sich mit mindestens 5%, bevorzugt mindestens 10%, besonders bevorzugt mindestens 25%, einer axialen Ausdehnung des Förderelements (6) überlappt.
3. Pumpe nach einem der Aspekte 1 oder 2, dadurch gekennzeichnet, dass der Auslassbereich (24) sich mit höchstens 75%, bevorzugt höchstens 65%, besonders bevorzugt höchstens 50%, einer axialen Ausdehnung des Förderelements (6) überlappt.
4. Pumpe nach einem der Aspekte 1 bis 3, dadurch gekennzeichnet, dass das Gehäuse (5) ein Gitter (19) umfasst, insbesondere im Auslassbereich (24).
5. Pumpe nach Aspekt 4, dadurch gekennzeichnet, dass das Gitter (19) ein Formgedächtnis-Material umfasst.
6. Pumpe nach einem der Aspekte 1 bis 5, dadurch gekennzeichnet, dass das Gehäuse (5) eine elastische Bespannung (21) aufweist.
7. Pumpe nach Aspekt 6, dadurch gekennzeichnet, dass der flüssigkeitsdichte Bereich(20) zumindest teilweise durch die elastische Bespannung (21) gebildet wird.
8. Pumpe nach einem der Aspekte 1 bis 7, dadurch gekennzeichnet, dass das Gehäuse (5) in einem expandierten Zustand im Auslassbereich (24) einen sich in Förderrichtung (26) verjüngenden konischen Abschnitt aufweist.
9. Pumpe nach Aspekt 8, dadurch gekennzeichnet, dass das Gehäuse (5) in einem expandierten Zustand im Auslassbereich (24) einen im Wesentlichen rohrförmigen Abschnitt aufweist, der an einem in Förderrichtung (26) gelegenen Ende mit dem konischen Abschnitt verbunden ist.
10. Pumpe nach einem der vorangehenden Aspekte, dadurch gekennzeichnet, dass das Gitter (19) in dem Auslassbereich (24) größere Gitteröffnungen als in dem flüssigkeitsdichten Bereich(20) aufweist.
11. Pumpe nach einem der Aspekte 1 bis 10, dadurch gekennzeichnet, dass ein Bereich des Auslassbereichs (24) von einem Abströmschirm (27) umgeben ist, der sich vom Gehäuse (5) in Förderrichtung (26) erstreckt.
12. Pumpe nach einem der Aspekte 1 bis 11, dadurch gekennzeichnet, dass ein Bereich des Auslassbereichs (24) von einem Abströmschlauch (27') umgeben ist, der sich vom Gehäuse (5) in Förderrichtung (26) erstreckt.
13. Pumpe nach einem der Aspekte 1 bis 12, dadurch gekennzeichnet, dass der Abströmschlauch (27') derart flexibel ausgebildet ist, dass dieser ein Rückschlagventil ausbildet.
14. Pumpe nach einem der Aspekte 1 bis 13, dadurch gekennzeichnet, dass der Einlassbereich (22) sich nicht mit einer axialen Ausdehnung des Förderelements (6) überlappt.
15. Pumpe nach einem der Aspekte 1 bis 14, dadurch gekennzeichnet, dass die Antriebswelle (3) an einem proximalen Ende der Antriebswelle (3) mit einem Motor (7) zum Antreiben der Antriebswelle (3) verbunden ist.
16. Pumpe nach einem der vorangehenden Aspekte, dadurch gekennzeichnet, dass die Welle (3) eine flexible Welle (3) ist.
17. Pumpe nach einem der vorangehenden Aspekte, dadurch gekennzeichnet, dass die Welle (3) in einem Katheter (2) geführt wird.
18. Pumpe nach einem der Aspekte 1 bis 17, dadurch gekennzeichnet, dass die Pumpe eingerichtet ist zum Pumpen von Blut von einem Ventrikel in ein Blutgefäß eines Patienten, wobei die Antriebswelle (3) in einem proximalen Bereich zum Anschließen an einen Motor (7) außerhalb eines Körpers des Patienten eingerichtet ist.
19. Pumpe nach einem der vorangehenden Aspekte, dadurch gekennzeichnet, dass das Gitter (19) in einem Bereich des Gehäuses (5) als Rautengitter mit im Wesentlichen rautenförmigen Gitteröffnungen ausgebildet ist.
20. Pumpe nach einem der vorangehenden Aspekte, dadurch gekennzeichnet, dass das Gitter (19) Gitterstreben (45) aufweist, wobei eine Anzahl der Gitterstreben (45) entlang eines Umfangs des Rautengitters m·2ⁿ mit natürlichen Zahlen m und n, vorzugsweise 32 oder 40, beträgt, wobei m größer als 2, vorzugsweise größer als 3, ist.
21. Pumpe nach Aspekt 20, dadurch gekennzeichnet, dass das Gehäuse (5) im Auslassbereich (24) entlang eines Umfangs m, vorzugsweise 4 oder 5, Gitterstreben (45) aufweist.
22. Pumpe, insbesondere Blutpumpe, umfassend eine in einer axialen Richtung verlaufende Antriebswelle (3), ein Förderelement (6), das in einem distalen Bereich der Antriebswelle (3) mit dieser verbunden ist, ein Gehäuse (5), welches das Förderelement (6) umgibt, wobei das Förderelement (6) und das Gehäuse (5) derart ausgebildet sind, dass diese sich nach einer aufgezwungenen Kompression selbstständig entfalten, und wobei das Gehäuse (5) ein Gitter (19), einen Einlassbereich (22) mit mindestens einer Einlassöffnung (23), einen flüssigkeitsdichten Bereich(20), der einen Bereich des Förderelements (6) umgibt, und einen Auslassbereich (24) mit mindestens einer Öffnung (23) zum Austritt eines Pumpmediums aufweist,
   dadurch gekennzeichnet, dass
   das Gitter (19) Gitteröffnungen und Gitterstreben (45) aufweist, wobei eine Anzahl der Gitterstreben (45) entlang eines Umfangs des Gitters (19) an einer ersten axialen Position des Gitters (19) m beträgt und wobei das Gitter (19) derart ausgeführt ist, dass sich die Anzahl der Gitterstreben (45) entlang eines Umfangs des Gitters (19) in einer axialen Richtung mit n Schritten auf m·2ⁿ Gitterstreben an einer zweiten axialen Position des Gitters (19) erhöht, wobei m und n natürlichen Zahlen sind, und m größer als 2, vorzugsweise größer als 3, ist.
23. Pumpe nach Aspekt 22, wobei die Anzahl der Gitterstreben (45) entlang des Umfangs des Gitters (19) an der zweiten axialen Position des Gitters (19) 32 oder 40 beträgt.
24. Pumpe nach Aspekt 23, dadurch gekennzeichnet, dass das Gehäuse (5) im Auslassbereich (24) entlang eines Umfangs m, vorzugsweise 4 oder 5, Gitterstreben (45) aufweist.

## Patentansprüche

1. Pumpe, insbesondere Blutpumpe, umfassend eine in einer axialen Richtung verlaufende Antriebswelle (3), ein Förderelement (6), das in einem distalen Bereich der Antriebswelle (3) mit dieser verbunden ist, ein Gehäuse (5), welches das Förderelement (6) umgibt, wobei das Förderelement (6) und das Gehäuse (5) derart ausgebildet sind, dass diese sich nach einer aufgezwungenen Kompression selbstständig entfalten, und wobei das Gehäuse (5) einen Einlassbereich (22) mit mindestens einer Einlassöffnung (23), einen flüssigkeitsdichten Bereich (20), der einen Bereich des Förderelements (6) umgibt, und einen Auslassbereich (24) mit mindestens einer Öffnung (23) zum Austritt eines Pumpmediums aufweist.

2. Pumpe nach Anspruch 1, **dadurch gekennzeichnet, dass** das Gehäuse (5) ein Gitter (19) umfasst.

3. Pumpe nach Anspruch 2, **dadurch gekennzeichnet, dass** das Gehäuse (5) eine elastische Bespannung auf einer Innenseite und auf einer Außenseite des Gitters (19) aufweist.

4. Pumpe nach einem der Ansprüche 2 oder 3, **dadurch gekennzeichnet, dass** das Gitter (19) ein Formgedächtnis-Material umfasst.

5. Pumpe nach einem der Ansprüche 1 bis 4, **dadurch gekennzeichnet, dass** das Gehäuse (5) in einem expandierten Zustand im Auslassbereich (24) einen sich in Förderrichtung (26) verjüngenden konischen Abschnitt aufweist.

6. Pumpe nach Anspruch 5, **dadurch gekennzeichnet, dass** das Gehäuse (5) in einem expandierten Zustand im Auslassbereich (24) einen im Wesentlichen rohrförmigen Abschnitt aufweist, der an einem in Förderrichtung (26) gelegenen Ende mit dem konischen Abschnitt verbunden ist.

7. Pumpe nach einem der vorangehenden Ansprüche, **dadurch gekennzeichnet, dass** das Gitter (19) in dem Auslassbereich (24) größere Gitteröffnungen als in dem flüssigkeitsdichten Bereich (20) aufweist.

8. Pumpe nach einem der Ansprüche 1 bis 7, **dadurch gekennzeichnet, dass** der Einlassbereich (22) sich nicht mit einer axialen Ausdehnung des Förderelements (6) überlappt.

9. Pumpe nach einem der Ansprüche 1 bis 8, **dadurch gekennzeichnet, dass** der Auslassbereich (24) sich mit mindestens 5%, bevorzugt mindestens 10%, besonders bevorzugt mindestens 25%, einer axialen Ausdehnung des Förderelements (6) überlappt.

10. Pumpe nach einem der Ansprüche 1 bis 14, **dadurch gekennzeichnet, dass** die Antriebswelle (3) an einem proximalen Ende der Antriebswelle (3) mit einem Motor (7) zum Antreiben der Antriebswelle (3) verbunden ist, wobei der Motor (7) zur Befestigung an einem Oberschenkel eines Patienten eingerichtet ist.

11. Pumpe nach einem der vorangehenden Ansprüche, **dadurch gekennzeichnet, dass** das Gitter (19) in einem Bereich des Gehäuses (5) als Rautengitter mit im Wesentlichen rautenförmigen Gitteröffnungen ausgebildet ist.

12. Pumpe nach einem der vorhergehenden Ansprüche, **dadurch gekennzeichnet, dass** das Gitter (19) Gitterstreben (45) aufweist, wobei alle Gitterstreben (45) entlang eines Umfangs des Gehäuses (5) an einer gewissen axialen Position in Form des Buchstabens Y paarweise zusammengeführt werden.

13. Pumpe nach einem der vorhergehenden Ansprüche, **dadurch gekennzeichnet, dass** die Gitterstreben (45) in konischen Bereichen des Auslassbereichs (24) und des Einlassbereichs (22) eine spiralförmige Struktur bilden.

14. Pumpe nach einem der vorhergehenden Ansprüche, **dadurch gekennzeichnet, dass** das Gehäuse (5) im Auslassbereich (24) entlang eines Umfangs 4 oder 5 Gitterstreben (45) aufweist.

15. Pumpe nach einem der vorhergehenden Ansprüche, **dadurch gekennzeichnet, dass** die Gitterstreben (45) in konischen Bereichen des Auslassbereichs (24) und des Einlassbereichs (22) eine spiralförmige Struktur bilden.
